# EUROPEAN PATENT SPECIFICATION

(11) **EP 2 068 963 B1**
(45) Date of publication and mention of the grant of the patent: **26.10.2011**
(21) Application number: 07842459.5
(22) Date of filing: 13.09.2007
(51) Int. Cl.: A61L 31/02, A61L 31/08, A61L 31/10, A61L 31/14, A61F 2/82

(54) **ENDOPROSTHESES**
ENDOPROTHESEN
ENDOPROSTHÈSES

(30) Priority: 18.09.2006 US 845478 P
(43) Date of publication of application: 17.06.2009
(73) Proprietor: Boston Scientific Limited, Christ Church (BB)
(72) Inventor: WEBER, Jan, 6228 GJ Maastricht (NL); ATANASOSKA, Liliana, Edina, Minnesota 55436 (US); LARSEN, Steven R., Lino Lakes, Minnesota 55110 (US); MERTENS, Steven P., Plymouth, Minnesota 55446 (US)
(74) Representative: Peterreins, Frank
(86) International application number: PCT/US2007/078429
(87) International publication number: WO 2008/036554

(56) References cited:
- EP-A- 0 966 979
- WO-A-2006/108065
- WO-A1-00/45744
- US-A1- 2003 033 007
- US-A1- 2005 283 229
- US-A1- 2006 193 892

## Description

### CROSS-REFERENCE TO RELATED APPLICATIONS

This application claims priority under 35 USC § 119(e) to U.S. Provisional Patent Application Serial No. 60/845,478, filed on September 18, 2006.

### TECHNICAL FIELD

This disclosure relates to endoprostheses, and to methods of making and delivering the same.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, e.g., so that it can contact the walls of the lumen.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn from the lumen.

U.S. Application 2006/0193892 discloses a medical device that is at least partially formed of a biodegradable polymer or microelectromechanical manufacturing (MEMS) technology, whereas the medical device can include one or more microstructures that are also formed by MEMS technology. The medical device can further include one or more biological agents that can be controllably and/or uncontrollably released from the medical device.

US 2006/0193892 A2 discusses the problem that fatigued portions of a stent formed of a biodegradable polymer can fracture and addresses it by modifying the properties of the biodegradable polymer. The coating can be used to control the time period that a portion of a medical device begins to dissolve. When a portion of the medical device microfactures, the material under the coating material is formulated to have a higher rate of dissolving, thus microfacture results in acceleration of the medical device to dissolve.

The feature of a coating of a stent that cracks upon expansion from the unexpanded state to the expanded state is described in WO 00/45744. The coating itself is preferably relatively inelastic so that the coating fractures during expansion of the boy portion.

### SUMMARY

This disclosure generally relates to endoprostheses that are, or that include portions that are, erodible or bioerodible as specified in the appendend claims.

In one aspect, the disclosure features an implantable endoprosthesis that includes a bioerodible body encapsulated in a protective coating. The protective coating prevents direct contact, at least for a time, between the bioerodible body and a bodily material. In another aspect, the disclosure features methods of making implantable endoprostheses. The methods include providing a bioerodible body and encapsulating the bioerodible body in a protective coating which prevents direct contact between the bioerodible body and a bodily material.

As an example, methods of delivering implantable endoprostheses are given. The methods include providing an implantable endoprosthesis that includes a bioerodible body encapsulated in a protective coating which prevents direct contact between the bioerodible body and a bodily material; delivering the implantable endoprosthesis to a site within a lumen; expanding the implantable endoprosthesis within the lumen; and disrupting the protective coating to allow direct contact between the bioerodible body and the bodily material.

Embodiments may include one or more of the following. The implantable endoprosthesis can be expandable, e.g., self-expandable, or non-expandable. The implantable endoprosthesis can be in the form of a stent.

The implantable endoprosthesis is expandable, and upon expansion from an unexpanded state to an expanded state, the protective coating thins to such an extent as to no longer prevent direct contact between the bioerodible body and the bodily material, or upon expansion from an unexpanded state to an expanded state, the protective coating cracks to such an extent as to no longer prevent direct contact between the bioerodible body and the bodily material. The bioerodible body can be, e.g., in the form of a tube that is circular in cross-section when viewed end-on along the longitudinal axis of the endoprosthesis.

The bioerodible body can be or can include a bioerodible metallic material, such as iron, magnesium, zinc, aluminum, calcium, or alloys of these metals, or the bioerodible body can be or can include a bioerodible polymeric material, such as polycaprolactone, polycaprolactone-polylactide copolymer, polycaprolactone-polyglycolide copolymer, polycaprolactone-polylactide-polyglycolide copolymer, polylactide, polycaprolactone-poly(β-hydroxybutyric acid) copolymer, poly(β-hydroxybutyric acid), or blends of these materials.

The protective coating can be or can include non-bioerodible material, such as a polymeric material or a ceramic. Examples of non-bioerodible polymeric materials include polycyclooctene, styrene-butadiene rubber, polyvinyl acetate, polyvinylidinefluoride, polymethylmethacrylate, polyurethane, polyethylene, polyvinyl chloride, and polyvinylidene dichloride, and examples of non-bioerodible ceramics include oxide of silicon (e.g., silicon dioxide) or oxides of titanium (e.g., titanium dioxide). The protective coating can also be, e.g., a carbonized polymeric material, such as diamond, e.g., amorphous diamond, or a diamond-like material.

The protective coating can be or can include a bioerodible polymeric material. In embodiments, the protective coating is formed from material from which the bioerodible body is made.

In particular embodiments, the bioerodible body is or includes a bioerodible metal, and the protective coating is or includes an oxide or a fluoride of the bioerodible metal. The protective coating can include a therapeutic agent, such as one that inhibits restenosis., e.g., paclitaxel, or a derivative thereof. The protective coating can be a single material or multiple materials, e.g., one material layer upon another material layer.

The endoprosthesis defines a plurality of spaced apart wells extending inwardly into to the endoprosthesis from an outer surface of the protective coating. Each well can be, e.g., substantially circular in cross-section when viewed from above. In such instances, each well can have an opening diameter of from about 2.5 µm to about 35 µm, e.g., from about 5 µm and 25 µm. In some embodiments, a spacing between wells is from about 10 µm to about 75 µm, e.g., from about 15 µm and 50 µm.

The disrupting can be performed during expansion. The disrupting can include piercing the protective coating. For example, the piercing can be performed during expansion on a balloon having an outer surface that includes projections which are configured to pierce the protective coating. Disruption can also occur before, during delivery, or after delivery. For example, the endoprosthesis, e.g. a self-expanding and held in a collapsed state, can be covered by a sheath during delivery. During deployment, as the sheath is withdrawn, the sheath can scratch or otherwise disrupt the protective coating.

Aspects and/or embodiments may have one or more of the following advantages. The endoprosthesis can be protected from premature erosion or damage such as during storage, handling and delivery. The endoprostheses can be configured to erode in a predetermined fashion and/or at a predetermined time after implantation into a subject, e.g., a human subject. For example, the predetermined manner of erosion can be from an inside of the endoprosthesis to an outside of the endoprosthesis, or from a first end of the endoprosthesis to a second end of the endoprosthesis. Many of the endoprostheses have portions which are protected from contact with bodily materials until it is desired for such portions to contact the bodily materials. The endoprostheses can exhibit a reduced likelihood of uncontrolled fragmentation, and the fragmentation can be controlled. The endoprostheses may not need to be removed from the body after implantation. Lumens implanted with such endoprostheses can exhibit reduced restenosis. The endoprostheses can have a low thrombogenecity. Some of the endoprostheses can be configured to deliver a therapeutic agent. Some of the endoprostheses have surfaces that support cellular growth (endothelialization).

An erodible or bioerodible endoprosthesis, e.g., a stent, refers to a device, or a portion thereof, that exhibits substantial mass or density reduction or chemical transformation, after it is introduced into a patient, e.g., a human patient. Mass reduction can occur by, e.g., dissolution of the material that forms the device and/or fragmenting of the device. Chemical transformation can include oxidation/reduction, hydrolysis, substitution, and/or addition reactions, or other chemical reactions of the material from which the device, or a portion thereof, is made. The erosion can be the result of a chemical and/or biological interaction of the device with the body environment, e.g., the body itself or body fluids, into which it is implanted and/or erosion can be triggered by applying a triggering influence, such as a chemical reactant or energy to the device, e.g., to increase a reaction rate. For example, a device, or a portion thereof, can be formed from an active metal, e.g., Mg or Ca or an alloy thereof, and which can erode by reaction with water, producing the corresponding metal oxide and hydrogen gas (a redox reaction). For example, a device, or a portion thereof, can be formed from an erodible or bioerodible polymer, or an alloy or blend erodible or bioerodible polymers which can erode by hydrolysis with water. The erosion occurs to a desirable extent in a time frame that can provide a therapeutic benefit. For example, in embodiments, the device exhibits substantial mass reduction after a period of time which a function of the device, such as support of the lumen wall or drug delivery is no longer needed or desirable. In particular embodiments, the device exhibits a mass reduction of about 10 percent or more, e.g. about 50 percent or more, after a period of implantation of one day or more, e.g. about 60 days or more, about 180 days or more, about 600 days or more, or 1000 days or less. In embodiments, the device exhibits fragmentation by erosion processes. The fragmentation occurs as, e.g., some regions of the device erode more rapidly than other regions. The faster eroding regions become weakened by more quickly eroding through the body of the endoprosthesis and fragment from the slower eroding regions. The faster eroding and slower eroding regions may be random or predefined. For example, faster eroding regions may be predefined by treating the regions to enhance chemical reactivity of the regions. Alternatively, regions may be treated to reduce erosion rates, e.g., by using coatings. In embodiments, only portions of the device exhibits erodibilty. For example, an exterior layer or coating may be erodible, while an interior layer or body is non-erodible. In embodiments, the endoprosthesis is formed from an erodible material dispersed within a non-erodible material such that after erosion, the device has increased porosity by erosion of the erodible material.

Erosion rates can be measured with a test device suspended in a stream of Ringer's solution flowing at a rate of 0.2 m/second. During testing, all surfaces of the test device can be exposed to the stream. For the purposes of this disclosure, Ringer's solution is a solution of recently boiled distilled water containing 8.6 gram sodium chloride, 0.3 gram potassium chloride, and 0.33 gram calcium chloride per liter.

All publications, patent applications, patents, and other references mentioned herein are incorporated by reference herein in their entirety.

Other aspects, features, and advantages will be apparent from the description and drawings, and from the claims.

### DESCRIPTION OF DRAWINGS

FIGS. 1A-1C are longitudinal cross-sectional views, illustrating delivery of a stent having a protective coating in a collapsed state (FIG. 1A); expansion of the stent (FIG. 1B); and deployment of the stent (FIG. 1C).
FIG. 2 is a transverse cross-sectional view of the unexpanded stent of FIG. 1A.
FIG. 3 is an enlarged side view of the balloon catheter shown in FIGS. 1A-1C, the balloon being in an expanded state.
FIG. 3A is an enlarged view of Region 3A of FIG. 3 illustrating the balloon wall in cross-section.
FIG 4 is a transverse cross-sectional view of the expanded stent shown in FIG. 1C, and illustrates a pierced coating.
FIG. 5A is a cross-sectional view of an unexpanded stent having a protective coating; while FIG. 5B is a cross-sectional view of the stent of FIG. 5A after expansion, illustrating thinning of the protective coating to expose the underlying stent body.
FIG. 6A is a cross-sectional view of an unexpanded stent having a protective coating; while FIG. 6B is a cross-sectional view of the stent of FIG. 6A after expansion, illustrating cracking of the protective coating to expose the underlying stent body.
FIG. 7 is a perspective view of a stent having a protective coating and defining a plurality of wells extending inwardly into the stent from an outer surface of the protective coating.
FIG. 7A is longitudinal cross-sectional view through a wall of the stent of FIG 7, taken along 7A-7A.
FIG. 7B is an enlarged top view of Region 7B of FIG. 7.
FIGS. 8A-8C are a series of cross-sectional views through the wall of the stent of FIG. 7 as the stent bioerodes.
FIG. 9 is a series of side views, showing manufacture of the stent of FIG. 7.

### DETAILED DESCRIPTION

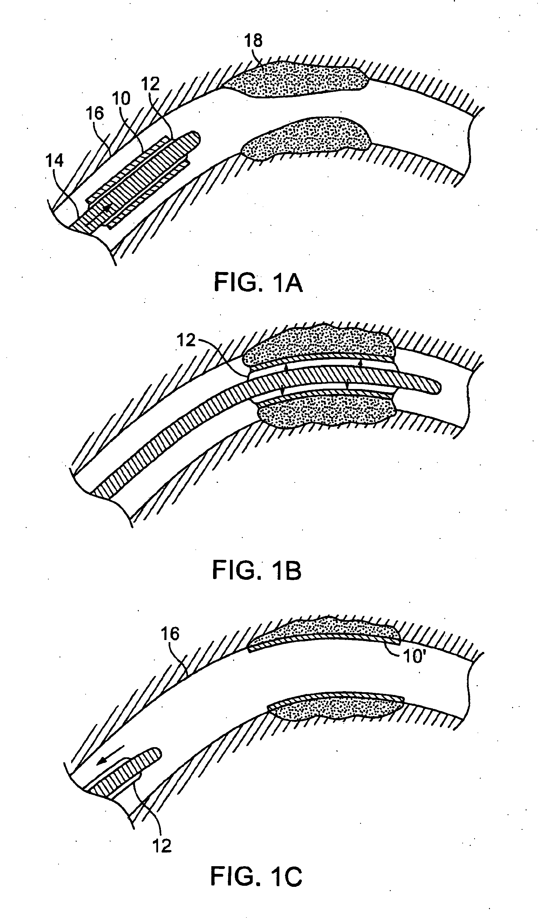

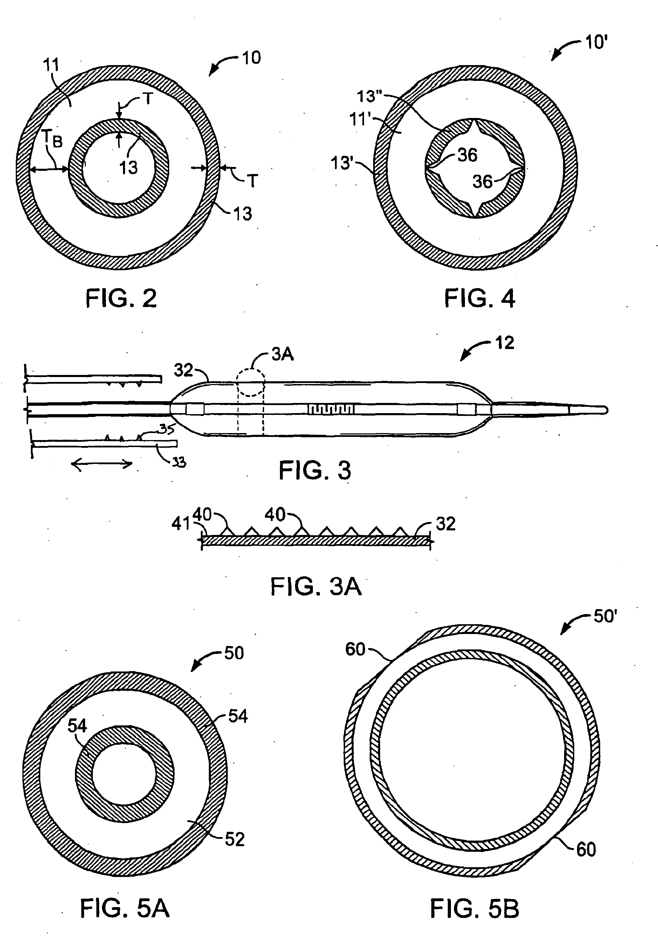

Referring to FIGS. 1A-1C and 2, a stent 10 includes a tubular bioerodible body 11 that is circular in transverse cross-section, and that is completely encapsulated in a protective coating 13, preventing direct exposure of any surface of the bioerodible body

11 and a bodily material, such as tissue or blood. Stent 10 is placed over a balloon 12 carried near a distal end of a catheter 14, and is directed through a lumen 16 (FIG. 1A) until the portion carrying the balloon 12 and stent 10 reaches the region of an occlusion 18. The stent 10 is then radially expanded by inflating the balloon 12 and compressed against the vessel wall with the result that occlusion 18 is compressed, and the vessel wall surrounding it undergoes a radial expansion (FIG. 1B). The pressure is then released from the balloon 12 and the catheter 14 is withdrawn from the vessel (FIG. 1C), leaving behind the expanded stent 10' in lumen 16.

Before deployment of the stent 10, during deployment of the stent, e.g., during expansion of the stent 10, or at a time after deployment, e.g., after expansion of the stent, the protective coating is disrupted, e.g., it is pierced, scratched, broken or eroded, to expose the bioerodible body 11 to body fluids to initiate erosion. The protective coating material and protective coating thickness T are chosen to provide a desired durability and/or disruption resistance, e.g., puncture resistance, preventing direct contact between the bioerodible body 11 and the bodily material for a desired time, such as the time required for implantation of the stent 10 into the body of a subject.

Referring now to FIGS. 1A-1C, 2, 3, 3A and 4, in a particular embodiment, unexpanded stent 10 is expanded on balloon 12 having wall 32. Wall 32 of balloon 12 has an outer surface 41 from which a plurality of projections 40 extend. Such projections 40 are configured to pierce, cut or scratch the protective inner coating during expansion of balloon 12, creating a plurality of breaches 36 that extend through inner coating 13". These breaches 36 allow bodily fluids such as blood to come into direct contact with the bioerodible body 11, initiating bioerosion. The balloon can include the projections 40 at predetermined locations that correspond to predetermined locations on stent 10. This allows the user to control how stent 10 will bioerode. For example, piercing the protective coatings only at one end can enable bioerosion of the stent from one longitudinal end to the other longitudinal end. Balloons with suitable projections include cutting balloons. Suitable balloons are described in O'Brien U.S. Patent No. 7,070,576 and Radisch, U.S. Patent No. 7,011,670. In addition or in the alternative, the delivery system can include a sheath 33 which covers the stent during delivery and is retracted to deploy the stent. The sheath can include cutting sections 35, e.g. metal projections embedded in a polymer sheath body, such that the projections breach the coating 13 on the outside of the stent as the sheath is retracted. By selecting the breaching mechanism, the coating can be breached on only the interior of the stent, only the exterior, or both the interior and the exterior. Stent delivery is further described in, for example, Wang U.S. 5,195,969, Hamlin U.S. 5,270,086, and Raeder-Devens, U.S. 6,726,712. Stents and stent delivery are also exemplified by the Radius® or Symbiot® systems, available from Boston Scientific Scimed, Maple Grove, MN.

Protective coating 13 can be bioerodible or non-bioerodible. When the protective coating 13 is bioerodible, it can be or can include a polymeric material, a metallic material (e.g., a metal or metal alloy) or a ceramic material. Examples of bioerodible polymers from which the protective coating 13 can be formed include polycaprolactone (PCL), polycaprolactone-polylactide copolymer (e.g., polycaprolactone-polylactide random copolymer), polycaprolactone-polyglycolide copolymer (e.g., polycaprolactone-polyglycolide random copolymer), polycaprolactone-polylactide-polyglycolide copolymer (e.g., polycaprolactone-polylactide-polyglycolide random copolymer), polylactide, polycaprolactone-poly(β-hydroxybutyric acid) copolymer (e.g., polycaprolactone-poly(β-hydroxybutyric acid) random copolymer) poly(β-hydroxybutyric acid) and mixtures of these polymers. Additional examples of bioerodible polymers are described by Sahatjian et. al. in U.S. Published Patent Application No. 2005/0251249.

Example of bioerodible metals or a metal alloys from which the protective coating 13 can be formed include iron, magnesium, zinc, aluminum and calcium. Examples of metallic alloys include iron alloys having, by weight, 88-99.8% iron, 0.1-7% chromium, 0-3.5% nickel, and less than 5% of other elements (e.g., magnesium and/or zinc); or 90-96% iron, 3-6% chromium and 0-3% nickel, plus 0-5% other metals. Other examples of alloys include magnesium alloys, such as, by weight, 50-98% magnesium, 0-40% lithium, 0-5% iron and less than 5% other metals or rare earths; or 79-97% magnesium, 2-5% aluminum, 0-12% lithium and 1-4% rare earths (such as cerium, lanthanum, neodymium and/or praseodymium); or 85-91 % magnesium, 6-12% lithium, 2% aluminum and 1% rare earths; or 86-97% magnesium, 0-8% lithium, 2-4% aluminum and 1-2% rare earths; or 8.5-9.5% aluminum, 0.15-0.4% manganese, 0.45-0.9% zinc and the remainder magnesium; or 4.5-5.3% aluminum, 0.28-0.5% manganese and the remainder magnesium; or 55-65% magnesium, 30-40% lithium and 0-5% other metals and/or rare earths. Magnesium alloys are available under the names AZ91 D, AM50A, and AE42, which are available from Magnesium-Elektron Corporation (United Kingdom). Other erodible metals or metal alloys are described by Bolz in U.S. 6,287,332 (e.g., zinc-titanium alloy and sodium-magnesium alloys); Heublein in U.S. Patent Application 2002/0004060; Kaese in Published U.S. Patent Application No. 2003/0221307; Stroganov in U.S. Patent No. 3,687,135; and Park in Science and Technology of Advanced Materials, 2, 73-78 (2001). Examples of bioerodible ceramics from which the protective coating 13 can be formed include beta-tertiary calcium phosphate (β-TCP), blends of β-TCP and hydroxy apatite, CaHPO₄ CaHPO₄-2H₂O, CaCO₃ and CaMg(CO₃)₂. Other bioerodible ceramics are discussed by Zimmermann in U.S. Patent No. 6,908,506, and Lee in U.S. Patent No. 6,953,594.

When the protective coating 13 is non-bioerodible, it can be or can include a polymeric material, a metallic material (e.g., a metal or metal alloy) or a ceramic material. Examples of non-bioerodible polymers from which the protective coating 13 can be formed include polycyclooctene (PCO), styrene-butadiene rubber, polyvinyl acetate, polyvinylidinefluoride (PVDF), polymethylmethacrylate (PMMA), polyurethanes, polyethylene, polyvinyl chloride (PVC), and blends thereof. Additional examples of non-bioerodible polymers are described by Sahatjian et. al. in U.S. Published Patent Application No. 2005/0251249. Examples of non-erodible metals and metal alloys from which the protective coating 13 can be formed include stainless steel, rhenium, molybdenum and molybdenum-rhenium alloy. Examples of non-bioerodible ceramics from which the protective coating 13 can be formed include oxides of silicon (e.g., silicon dioxide), oxides of titanium (e.g., titanium dioxide) or oxides of zirconium (e.g., zirconium dioxide).

The protective coating can also be, e.g., a carbonized polymeric material, such as diamond, e.g., amorphous diamond, or a diamond-like material. Such carbonized materials are described by Weber et al. in MEDICAL BALLOONS AND METHODS OF MAKING THE SAME, U.S. Patent Application Serial Nos. 11/355,392, filed February 16, 2006, and BIOERODIBLE ENDOPROSTHESES AND METHODS OF MAKING THE SAME, U.S. Patent Application Serial No. 11/355,368, filed February 16, 2006.

In particular embodiments, the protective coating 13 is formed from the material from which the bioerodible body 11 is made. For example, the body can be magnesium or a magnesium alloy, and the protective coating 13 can be made by ion implanting oxygen or nitrogen into the bioerodible body 11. During such an implantation, the oxygen or nitrogen reacts with the magnesium of the body 11, to produce a protective coating. As another example, the body can be magnesium or a magnesium alloy, and the protective coating 13 can be made by treating the bioerodible body 11 with hydrogen fluoride. During such a treatment, the hydrogen fluoride reacts with the magnesium of the body 11, to produce a magnesium fluoride protective coating.

In particular embodiments, the protective coating 13 is formed integrally on top of a bioerodible body 11. For example, the body can be magnesium or a magnesium alloy, and the protective coating 13 can be a deposited coating, e.g., deposited using chemical vapor deposition. For example, silicon dioxide, titanium dioxide or zirconium dioxide can be deposited in this fashion.

In embodiments, the protective coating 13 is a bioerodible polymeric material, having thickness T of, e.g., between about 0.1 µm and 100 µm, e.g., between about 1 µm and 50 µm, or between about 5 µm and 35 µm. In embodiments, the protective coating 13 is a bioerodible metallic material or ceramic material, having thickness T of the coating, e.g., between about 0.01 µm and 10 µm, e.g., between about 0.05 µm and 7.5 µm, or between about 0.1 µm and 5 µm. In embodiments, the protective coating 13 is a non-bioerodible polymeric material, having thickness T of the coating, e.g., between about 0.5 µm and 50 µm, e.g., between about 1 µm and 25 µm, or between about 2 µm and 20 µm. In embodiments in which the protective coating 13 is a non-bioerodible metallic material or ceramic material, the thickness T of the coating can be, e.g., between about 0.01 µm and 5 µm, e.g., between about 0.05 µm and 5 µm, or between about 0.1 µm and 2 µm. As used herein, "metallic material" means a pure metal, a metal alloy or a metal composite. In embodiments, a protective coating prevents direct contact between Ringer's test solution and the bioerodible body for at least 6 hours upon immersion in the Ringer's solution at 25 °C.

In some embodiments, the protective coating 104 is formed of a bioerodible material that erodes at a slower rate than body 102 material, e.g., less than 50 percent of the rate of the body material, less than 35 percent, less than 20 percent, less than 15 percent, less than 10 percent, less than 5 percent, less than 2.5 percent, or even less than 1 percent of the rate of the body material.

The protective coating 13 can be made by a variety of techniques including dip coating, spray coating, ion implantation (e.g., plasma immersion ion implantation), pulsed laser deposition, laser treatment, physical vapor deposition (e.g., sputtering), chemical vapor deposition, vacuum arc deposition, electrochemical plating, chemical treatment, powder coating, painting, electrocoating, sol-gel coating and polymer plating (e.g., plasma polymerization). Plasma immersion ion implantation (PIII) is described by Weber et al. in MEDICAL BALLOONS AND METHODS OF MAKING THE SAME, U.S. Patent Application Serial Nos. 11/355,392, filed February 16, 2006, and BIOERODIBLE ENDOPROSTHESES AND METHODS OF MAKING THE SAME, U.S. Patent Application Serial No. 11/355,368, filed February 16, 2006; by Chu in U.S. Patent No. 6,120,660; and by Brukner and Kutsenko in Acta Materialia, 52, 4329-4335 (2004). Pulsed laser deposition is described by Wang et al. in Thin Solid Films, 471, 86-90 (2005); protective coatings on magnesium are reviewed by Gray et al. in Journal of Alloys and Compounds, 336, 88-113 (2002); and vacuum arc deposition is described by Straumal et al. in Thin Solid Films, 383, 224-226 (2001).

The body material and thickness T_{B} are chosen to provide a desired mechanical strength and a desired bioerosion rate. The bioerodible body 11 can be or can include a bioerodible polymeric material, a bioerodible metallic material (e.g., a metal or metal alloy), or a bioerodible ceramic material. The bioerodible polymeric material, metallic material, or ceramic material can be, e.g., any of the bioerodible materials described above. In embodiments in which the bioerodible body 11 is formed from a bioerodible polymeric material, the transverse thickness T_{B} can be, e.g., between about 0.5 mm and about 5.0 mm, e.g., between about 0.5 mm and 3.0 mm, or between about 1 mm and 2.5 mm. In embodiments in which the bioerodible body 11 is formed from a bioerodible metallic material or ceramic material, the transverse thickness T_{B} can be, e.g., between about 0.1 mm and about 2.5 mm, e.g., between about 0.25 mm and 2.0 mm, or between about 0.3 mm and 1.5 mm.

Any of the metallic materials, ceramic materials, or polymeric materials used to form the bioerodible body 11 or protective coating 13 can be made porous. For example, a porous metal material can be made by sintering metal particles, e.g., having diameters between about 0.01 micron and 20 micron, to form a porous material having small (e.g., from about 0.05 to about 0.5 micron) and large (e.g., from about 1 micron to about 10 micron) interconnected voids though which a fluid may flow. The voids in the porous material can be, e.g., used as depositories for a therapeutic agent that has been intercalated into the porous material. Such porous materials can have a total porosity, as measured using mercury porosimetry, of from about 80 to about 99 percent, e.g., from about 80 to about 95 percent or from about 85 to about 92 percent, and a specific surface area, as measured using BET (Brunauer, Emmet and Teller), of from about 200 cm²/cm³ to about 10,000 cm²/cm³, e.g., from about 250 cm²/cm³ to about 5,000 cm²/cm³ or from about 400 cm²/cm³ to about 1,000 cm²/cm³. When bioerodible materials are utilized, the porous nature of the material can aid in the erosion of the material, as least in part, due to its increased surface area. In addition, when bioerodible materials are utilized, the porosity of the materials can ensure small fragment sizes. Porous materials and methods of making porous materials are described by Date et al. in U.S. Patent No. 6,964,817; by Hoshino et al. in U.S. Patent No. 6,117,592; and by Sterzel et al. in U.S. Patent No. 5,976,454.

Referring now to FIGS. 5A and 5B, in another embodiment, a stent 50 includes a tubular bioerodible body 52 that is circular in transverse cross-section, and that is completely encapsulated in a protective coating 54, preventing direct contact between any surface of the bioerodible body 52 and a bodily material. Upon expansion within a lumen to an expanded stent 50', the protective coating thins to such an extent to create breaches 60. At the breaches 60, the protective coating no longer prevents direct contact between the bioerodible body and the bodily material. Such breaches allow bodily fluids to come into direct contact with the bioerodible body to initiate bioerosion. The breeches can occur randomly or can be formed at select locations by, e.g., providing reduced thickness regions in the coating.

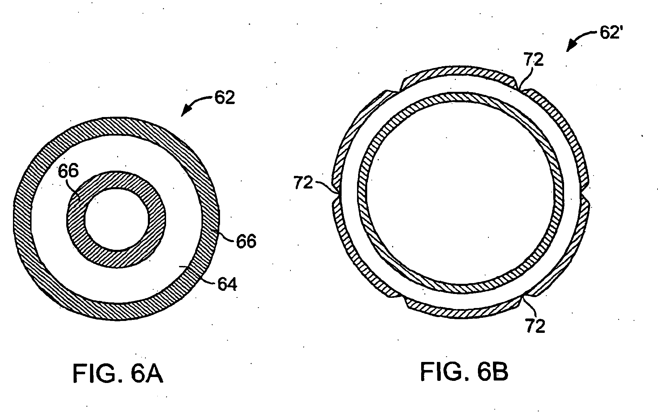

Referring now to FIGS. 6A and 6B, in yet another embodiment, a stent 62 includes a tubular bioerodible body 64 that is circular in transverse cross-section, and that is completely encapsulated in a protective coating 66. Upon expansion within a lumen to expanded stent 62', the protective coating cracks, e.g., because its ability to deform and stretch is less than that of the bioerodible body 64, creating breaches 72 in the protective coating. The breaches allow for direct contact between the bioerodible body and the bodily material, initiating bioerosion at these sites. The cracking can occur randomly or can be formed at select locations, e.g., by making the coating stiffer or more brittle at select locations such as by crosslinking of the coating at select locations.

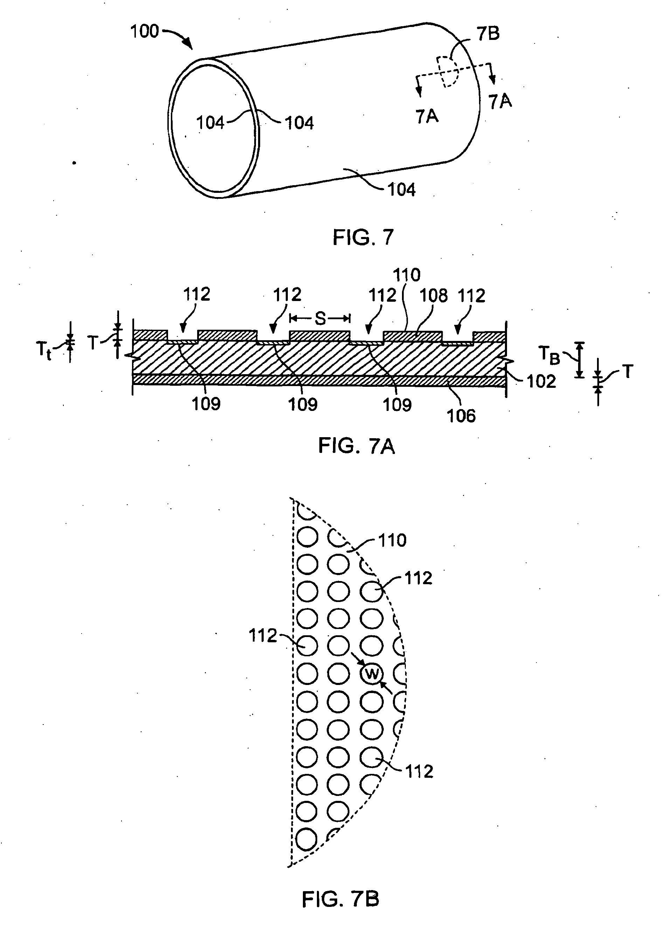

Referring now to FIG. 7, 7A and 7B, a stent 100 includes a tubular bioerodible body 102 that is circular in transverse cross-section, and that is completely encapsulated in a protective coating 104, preventing direct contact between any surface of the bioerodible body 102 and a bodily material. The stent 100 defines a plurality of spaced apart wells 112 which extend inwardly into the stent from an outer surface 110 of the outer protective coating 108. A bottom of each well correspond to thin regions 109 of the outer protective coating 108. The thin regions 109 represent near breaches or "weak portions" in the protective coating encapsulating the stent body. In the particular embodiment shown, inner protective coating 106 has a constant longitudinal thickness across the stent.

The protective coating material, nominal protective coating thickness T and the protective coating thickness Tₜ in thin regions 109 are chosen such that the protective coating prevents direct contact between the bioerodible body and a bodily material for a desired time as described above. In some embodiments, protective coating thickness Tₜ in thin regions 109 is from about 2 percent to about 75 of the nominal coating thickness T, e.g., from about 5 percent to about 50 percent of the nominal thickness, or from about 7.5 percent to about 25 percent of the nominal thickness. Spacing S between adjacent wells and the opening width W of wells are chosen such that the stent 100 erodes in a desired manner at a desired rate. For example, the width W is such that a bodily fluid can flow into the well. For example, the opening width W, e.g., the diameter of the opening in the embodiment shown, can be from about 2.5 µm to about 35 µm, e.g., from about 3 µm to about 25 µm, or from about 5 µm to about 15 µm. The spacing S between adjacent wells 112 is, e.g., from about 7.5 µm to about 150 µm, e.g., from about 9 µm to about 100 µm, or from about 10 µm to about 75 µm.

In some embodiments, during expansion of stent 100 on a balloon, the thin regions 109 become even thinner and breach, allowing bodily fluids to come into direct contact with the bioerodible body, initiating erosion.

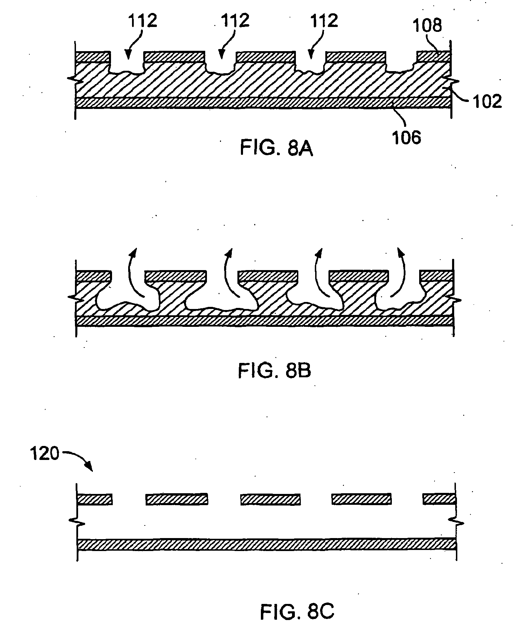

Erosion of the stent in FIG. 7 is illustrated when the coating 104 is made of a non-bioerodible material. Referring now to FIGS. 8A-8C, after breach of thin regions 109 of protective coating 104, e.g., by expanding to breach the thin regions, bodily fluids come into direct contact with body 102 by entering wells 112, initiating bioerosion of the stent. Since in this particular embodiment the protective coating is made of a non-bioerodble material, as bioerosion progresses, only the bioerodible body 102 erodes, leaving behind an empty shell 120 that is, e.g., completely encapsulated by cell growth. Having the stent degrade in this manner reduces the probability of uncontrolled fragmentation or having large fragmentation pieces becoming unattached from the bulk stent and entering the blood stream.

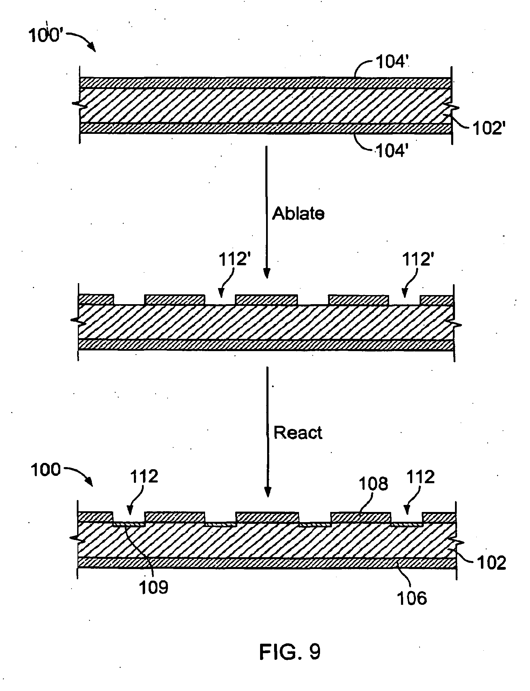

Referring now to FIGS. 9, stent 100 can be prepared from pre-stent 100. Pre-stent 100' includes a bioerodible body 102' that includes a bioerodible material such as a metallic material (e.g., magnesium), that is completely encapsulated in a protective coating 104' such as a metallic oxide or fluoride (e.g., magnesium fluoride). The coating can be placed or deposited on body 102' by any of the methods described above. Breaches 112' are cut into the outer protective coating, e.g., by laser ablation, and then thin regions 109 are created by, e.g., using the same material as used to form the coating 104', or a different material. For example, when the bioerodible material of the body is magnesium, thin regions 109 can be formed by dipping the pre-stent in an aqueous solution of hydrogen fluoride or by exposing the pre-stent to hydrogen fluoride gas. The hydrogen fluoride reacts with the magnesium, forming magnesium fluoride.

If desired, the protective coating can include a therapeutic agent dispersed therein and/or thereon. The therapeutic agent can be a genetic therapeutic agent, a non-genetic therapeutic agent, or cells. Therapeutic agents can be used singularly, or in combination. Therapeutic agents can be, e.g., nonionic, or they may be anionic and/or cationic in nature. A preferred therapeutic agent is one that inhibits restenosis. A specific example of one such therapeutic agent that inhibits restenosis is paclitaxel or derivatives thereof, e.g., docetaxel. Soluble paclitaxel derivatives can be made by tethering solubilizing moieties off the 2' hydroxyl group of paclitaxel, such as -COCH₂CH₂CONHCH₂CH₂(OCH₂)ₙOCH₃ (n being, e.g., 1 to about 100 or more). Li et al., U.S. Patent No. 6,730,699 describes additional water soluble derivatives of paclitaxel. 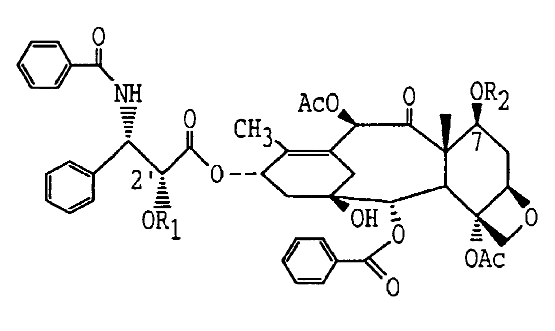 Paclitaxel: R1=R2=H

Exemplary non-genetic therapeutic agents include: (a) anti-thrombotic agents such as heparin, heparin derivatives, urokinase, PPack (dextrophenylalanine proline arginine chloromethylketone), and tyrosine; (b) anti-inflammatory agents, including non-steroidal anti-inflammatory agents (NSAID), such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, rapamycin (sirolimus), biolimus, tacrolimus, everolimus, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, antiplatelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines, (r) hormones; and (s) antispasmodic agents, such as alibendol, ambucetamide, aminopromazine, apoatropine, bevonium methyl sulfate, bietamiverine, butaverine, butropium bromide, n-butylscopolammonium bromide, caroverine, cimetropium bromide, cinnamedrine, clebopride, coniine hydrobromide, coniine hydrochloride, cyclonium iodide, difemerine, diisopromine, dioxaphetyl butyrate, diponium bromide, drofenine, emepronium bromide, ethaverine, feclemine, fenalamide, fenoverine, fenpiprane, fenpiverinium bromide, fentonium bromide, flavoxate, flopropione, gluconic acid, guaiactamine, hydramitrazine, hymecromone, leiopyrrole, mebeverine, moxaverine, nafiverine, octamylamine, octaverine, oxybutynin chloride, pentapiperide, phenamacide hydrochloride, phloroglucinol, pinaverium bromide, piperilate, pipoxolan hydrochloride, pramiverin, prifinium bromide, properidine, propivane, propyromazine, prozapine, racefemine, rociverine, spasmolytol, stilonium iodide, sultroponium, tiemonium iodide, tiquizium bromide, tiropramide, trepibutone, tricromyl, trifolium, trimebutine, tropenzile, trospium chloride, xenytropium bromide, ketorolac, and pharmaceutically acceptable salts thereof.

Exemplary genetic therapeutic agents include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or micro particles, with and without targeting sequences such as the protein transduction domain (PTD).

Cells for use include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

The stents described herein can be delivered to a desired site in the body by a number of catheter delivery systems, such as a balloon catheter system, as described above. Exemplary catheter systems are described in U.S. Patent Nos. 5,195,969, 5,270,086, and 6,726,712. The Radius^{®} and Symbiot^{®} systems, available from Boston Scientific Scimed, Maple Grove, MN, also exemplify catheter delivery systems. The stents described herein can be configured for vascular e.g. coronary or non-vascular lumens. For example, they can be configured for use in the esophagus or the prostate. Other lumens include biliary lumens, hepatic lumens, pancreatic lumens, uretheral lumens and ureteral lumens. Any stent described herein can be dyed or rendered radio-opaque by addition of, e.g., radio-opaque materials such as barium sulfate, platinum or gold, or by coating with a radio-opaque material.

While stents have been shown, other endoprostheses are possible. For example, the endoprosthesis can be in the form of a stent-graft or a filter.

While embodiments have been shown in which the bioerodible body is in the form of a tube that is circular in cross-section when viewed end-on along the longitudinal axis of the stent (e.g., FIG. 2), the tube can have a non-circular cross-section. For example, the tube can be square, rectangular, hexagonal, or octagonal when viewed end-on along the longitudinal axis of the stent.

While stents have been shown that include a bioerodible tubular member having a constant longitudinal transverse thickness, in some embodiments, the thickness is not constant. For example, the thickness can continuously thin from a proximal end of the bioerodible body to a distal end of the bioerodible body. Such embodiments can be advantageous when it is desirable to have the stent erode from one end to the other. While stents have been shown that have an equal coating thickness on both the inside and outside of the tubular structure (e.g., FIG. 2), in some embodiments, the protective coating thickness on the inside is thinner than the protective coating thickness on the outside of the tubular structure. Such embodiments can be advantageous when it is desirable to have the stent erode from the inside towards the outside of the stent. In addition, while embodiments, have been shown (e.g., FIG. 2 and FIG. 7A) in which the protective coating has a substantially constant thickness along a longitudinal portion of the stent, in some embodiments, the protective coating varies along a longitudinal length of the stent, e.g., by 10 percent, 20 percent or even 50 percent. For example, the thickness can continuously thin from a proximal end of the stent to a distal end of the stent. Such embodiments can be advantageous when it is desirable to have the stent erode from one end to the other.

While protective coatings have been described that include a single material, in some embodiments, multiple materials form the protective coating. For example, the protective coating can be a blend of two or more materials, or the protective coating can be two or more layers of materials, with each layer being a different material.

In embodiments, a coating that does not encapsulate the body can be breached by the techniques described herein. For example, the coating may be provided only on the interior or exterior surface of the stent. In embodiments, the coatings can be scratched or abraded at select locations manually or with a tool, e.g. a blade, prior to delivery in the body. In embodiments, the coating can be modified, e.g. scratched or punctured as described above, so that the coating is not entirely breached but its thickness is reduced in the modified region.

Still other embodiments are within the scope of the following claims.

## Claims

1. An implantable endoprosthesis comprising a bioerodible body and a protective coating, the protective coating preventing direct contact between the bioerodible body and a bodily material when the endoprosthesis is in a body and in an unexpanded state, wherein the protective coating is designed to thin and create breaches at regions with reduced thickness or to crack at more brittle locations, upon expansion from the unexpanded state to an expanded state, to such an extent as to no longer prevent direct contact between the bioerodible body and the bodily material when implanted.

2. The implantable endoprosthesis of claim 1, wherein the bioerodible body is in the form of a tube.

3. The implantable endoprosthesis of one of the preceding claims, wherein the bioerodible body comprises a bioerodible metallic material selected from the group consisting of iron, magnesium, zinc, aluminum, calcium, and alloys thereof.

4. The implantable endoprosthesis of one of the preceding claims, wherein the bioerodible body comprises a bioerodible polymeric material.

5. The implantable endoprosthesis of one of the preceding claims, wherein the protective coating comprises non-bioerodible material.

6. The implantable endoprosthesis of claim 5, wherein the non-bioerodible material is a polymeric material or a ceramic.

7. The implantable endoprosthesis of claim 6, wherein the non-bioerodable material is a ceramic comprising an oxide of silicon or an oxide of titanium.

8. The implantable endoprosthesis of one of the preceding claims, wherein the protective coating comprises a bioerodible polymeric material.

9. The implantable endoprosthesis of one of the preceding claims, wherein the bioerodible body comprises a bioerodible metal, and wherein the protective coating comprises an oxide or fluoride of the bioerodible metal.

10. The implantable endoprosthesis of one of the preceding claims, wherein the protective coating includes a therapeutic agent.

11. The implantable endoprosthesis of one of the preceding claims, wherein the protective coating varies in thickness by more than 10 % along a longitudinal length of the endoprosthesis.

12. The implantable endoprosthesis of one of the preceding claims, wherein the endoprosthesis defines a plurality of spaced apart wells extending inwardly to the endoprosthesis from an outer surface of the protective coating.

13. The implantable endoprosthesis of claim 12, wherein each well has an opening diameter of from about 2.5 µm to about 35 µm, and wherein a spacing between wells is from about 10 µm to about 75 µm.

## Patentansprüche

1. Eine implantierbare Endoprothese umfassend einen bioerodierbaren Körper und eine Schutzschicht, wobei die Schutzschicht direkten Kontakt zwischen dem bioerodierbaren Körper und einem Körpermaterial verhindert, wenn die Endoprothese in einem Körper und in einem nicht-expandierten Zustand ist, wobei die Schutzschicht ausgelegt ist, beim Expandieren von einem nicht-expandierten Zustand in einen expandierten Zustand an Regionen mit verringerter Dicke dünn zu werden und Brüche zu bilden, oder an spröderen Stellen zu brechen, zu einem Ausmaß, dass ein direkter Kontakt zwischen dem bioerodierbaren Körper und dem Körpermaterial nach Implantieren nicht mehr verhindert wird.

2. Die implantierbare Endoprothese aus Anspruch 1, wobei der bioerodierbare Körper in der Form einer Röhre ist.

3. Die implantierbare Endoprothese aus einem der vorangehenden Ansprüche, wobei der bioerodierbare Körper ein bioerodierbares metallisches Material ausgewählt aus der Gruppe bestehend aus Eisen, Magnesium, Zink, Aluminium, Kalzium, und Legierungen daraus, umfasst.

4. Die implantierbare Endoprothese aus einem der vorangehenden Ansprüche, wobei der bioerodierbare Körper ein bioerodierbares polymeres Material umfasst.

5. Die implantierbare Endoprothese aus einem der vorangehenden Ansprüche, wobei die Schutzschicht ein nicht-bioerodierbares Material umfasst.

6. Die implantierbare Endoprothese aus Anspruch 5, wobei das nichtbioerodierbare Material ein polymeres Material oder eine Keramik ist.

7. Die implantierbare Endoprothese aus Anspruch 6, wobei das nichtbioerodierbare Material eine Keramik, umfassend ein Siliziumoxid oder ein Titanoxid, ist.

8. Die implantierbare Endoprothese aus einem der vorangehenden Ansprüche, wobei die Schutzschicht ein bioerodierbares polymeres Material umfasst.

9. Die implantierbare Endoprothese aus einem der vorangehenden Ansprüche, wobei der bioerodierbare Körper ein bioerodierbares Metall umfasst, und wobei die Schutzschicht ein Oxid oder Fluorid des bioerodierbaren Metalles umfasst.

10. Die implantierbare Endoprothese aus einem der vorangehenden Ansprüche, wobei die Schutzschicht einen therapeutischen Wirkstoff beinhaltet.

11. Die implantierbare Endoprothese aus einem der vorangehenden Ansprüche, wobei die Schutzschicht in der Dicke um mehr als 10 % entlang einer longitudinalen Länge der Endoprothese variiert.

12. Die implantierbare Endoprothese aus einem der vorangehenden Ansprüche, wobei die Endoprothese eine Mehrzahl von räumlich getrennten Löchern definiert, die sich einwärts in die Endoprothese von einer äußeren Oberfläche der Schutzschicht erstrecken.

13. Die implantierbare Endoprothese aus Anspruch 12, wobei jedes Loch einen Öffnungsdurchmesser von etwa 2,5 µm bis etwa 35 µm hat, und wobei ein Abstand zwischen den Löchern von etwa 10 µm bis etwa 75 µm ist.

## Revendications

1. Endoprothèse implantable comprenant un corps bioérodable et un revêtement protecteur, le revêtement protecteur empêchant le contact direct entre le corps bioérodable et une matière corporelle lorsque l'endoprothèse est dans un corps et à l'état non déployé, dans laquelle le revêtement protecteur est conçu pour diminuer d'épaisseur et créer des brèches au niveau de régions d'épaisseur réduite ou pour se fissurer au niveau d'endroits plus fragiles, lors du déploiement de l'état non déployé à un état déployé, au point de ne plus empêcher le contact direct entre le corps bioérodable et la matière corporelle lorsqu'elle est implantée.

2. Endoprothèse implantable selon la revendication 1, dans laquelle le corps bioérodable est sous la forme d'un tube.

3. Endoprothèse implantable selon l'une quelconque des revendications précédentes, dans laquelle le corps bioérodable comprend un matériau métallique bioérodable choisi dans le groupe constitué par le fer, le magnésium, le zinc, l'aluminium, le calcium et les alliages de ceux-ci.

4. Endoprothèse implantable selon l'une quelconque des revendications précédentes, dans laquelle le corps bioérodable comprend un matériau polymère bioérodable.

5. Endoprothèse implantable selon l'une quelconque des revendications précédentes, dans laquelle le revêtement protecteur comprend un matériau non bioérodable.

6. Endoprothèse implantable selon la revendication 5, dans laquelle le matériau non bioérodable est un matériau polymère ou une céramique.

7. Endoprothèse implantable selon la revendication 6, dans laquelle le matériau non bioérodable est une céramique comprenant un oxyde de silicium ou un oxyde de titane.

8. Endoprothèse implantable selon l'une quelconque des revendications précédentes, dans laquelle le revêtement protecteur comprend un matériau polymère bioérodable.

9. Endoprothèse implantable selon l'une quelconque des revendications précédentes, dans laquelle le corps bioérodable comprend un métal bioérodable et dans laquelle le revêtement protecteur comprend un oxyde ou fluorure du métal bioérodable.

10. Endoprothèse implantable selon l'une quelconque des revendications précédentes, dans laquelle le revêtement protecteur comprend un agent thérapeutique.

11. Endoprothèse implantable selon l'une quelconque des revendications précédentes, dans laquelle le revêtement protecteur varie en termes d'épaisseur de plus de 10 % le long d'une longueur dans la direction longitudinale de l'endoprothèse.

12. Endoprothèse implantable selon l'une quelconque des revendications précédentes, l'endoprothèse délimitant une pluralité de puits espacés les uns des autres s'étendant vers l'intérieur de l'endoprothèse à partir d'une surface extérieure du revêtement protecteur.

13. Endoprothèse implantable selon la revendication 12, dans laquelle chaque puits a un diamètre d'ouverture d'environ 2,5 µm à environ 35 µm et dans laquelle l'espacement entre des puits est d'environ 10 µm à environ 75 µm.
